Europäisches Patentamt

European Patent Office

Office européen des brevets

(11) Veröffentlichungsnummer: **0 302 278**
**A1**

(12) **EUROPÄISCHE PATENTANMELDUNG**

(21) Anmeldenummer: **88111314.6**

(22) Anmeldetag: **14.07.88**

(51) Int. Cl.⁴: **A61M 5/20**

(30) Priorität: **05.08.87 DD 305714**

(43) Veröffentlichungstag der Anmeldung:
**08.02.89 Patentblatt 89/06**

(84) Benannte Vertragsstaaten:
**AT BE CH DE FR GB IT LI NL SE**

(71) Anmelder: **VEB Transformatoren- und Röntgenwerk "Hermann Matern"**
**Overbeckstrasse 48**
**DDR-8030 Dresden(DD)**

(72) Erfinder: **Haufe, Dietrich, Dipl.-Ing.**
**Burgwartstrasse 47**
**DDR-8028 Dresden(DD)**
Erfinder: **Roszat, Johannes**
**Thäterstrasse 5**
**DDR-8030 Dresden(DD)**

(74) Vertreter: **Patentanwälte Beetz sen. - Beetz jun. Timpe - Siegfried - Schmitt-Fumian-Mayr**
**Steinsdorfstrasse 10**
**D-8000 München 22(DE)**

(54) **Injektionseinrichtung.**

(57) Die Erfindung betrifft eine Injektionseinrichtung für die automatische Injektion von Pharmaka, z.B. für Diabetiker oder für andere spezielle Anwendungsgebiete. Die Injektionseinrichtung weist einen Antrieb 10, 12 und eine Vibrationseinrichtung 22 auf; der Anrieb 10, 12 steht einerseits mit einem Kolbenschieber 15 und andererseits über eine schaltbare Kupplung 16 mit einem axial beweglichen, die Kanülen 6 freigebenden Mundstück 8 in Verbindung. Die Vibrationseinrichtung 22 ist mit der Kanülenaufnahme 5 verbunden und überlagert zumindest während des Einstiches den Kanülen 6 Axialschwingungen, wobei zwischen Spritzenbehälter 2 und Kanülenaufnahme 5 ein elastisches, die Vibration nicht übertragendes Glied 4 vorgesehen ist.

EP 0 302 278 A1

## Injektionseinrichtung

Die Erfindung betrifft eine Injektionseinrichtung für die automatische Injektion insbesondere von Pharmaka, z.B. für Diabetiker oder für andere spezielle Anwendungsgebiete.

Herkömmliche Injektionsspritzen, die von medizinischem Personal und auch im besonderen von Patienten selbst benutzt werden können, besitzen einen Kolben, der von Hand bewegbar ist, um durch eine Kanüle das Pharmakon aufzuziehen und durch Gegenbewegung nach Einstich in der Körper das Pharmakon zu injizieren. Diese Spritzen haben den Nachteil, daß der Einstich für den Patienten mehr oder weniger schmerzhaft ist.

Des weiteren sind die sogenannten selbsttätigen Injektionseinrichtungen bekannt, die Antriebsmittel für die Bewegung von Kanüle und Spritzenkolben besitzen. So ist eine automatische Injektionseinrichtung bekannt, die aus einer auswechselbaren, auf einer Wiege befindlichen Injektionsspritze besteht, wobei zu den genannten Bewegungen elektrische, mechanische, elektromagnetische oder pneumatische Antriebsmittel verwendet werden (DD-A1-132 169).

Der Kolbenhub und die Einstichtiefe werden mittels Anschlägen begrenzt. Der Einstich der Kanüle ist jedoch auch bei diesen Injektionseinrichtungen schmerzhaft, insbesondere, weil er stoßartig, ausgelöst durch einen Elektromagneten, erfolgt. Schon aus diesem Grunde wäre die Injektionseinrichtung beispielsweise für den speziellen Anwendungsfall der Penis-Stimulation gänzlich ungeeignet.

Ferner ist es für das Einbringen von drahtförmigen Sonden in tiefliegende Bereiche des Körpers bekannt, das angespitzte Ende einer Sonde axial schwingend mit sehr geringem Druck einzubringen (DD-A1-22 029). Die Schwingungen werden von einem Ultraschallschwinger erzeugt. Diese Einrichtung ist mit einem hohen apparativen Aufwand verbunden, ermöglicht keinen definierten Axialvorschub und ist nicht zur Injektion von Flüssigkeiten vorgesehen bzw. verwendbar.

Zur schmerzarmen Injektion wurde ferner bereits eine Injektionseinrichtung angegeben, bei der die Kanüle während des Einstechens in axiale Schwingungen im Frequenzbereich von 40 bis 500 Hz versetzt wird. Die Injektionseinrichtung besteht aus einem Antrieb für den axialen Vorschub und die Bewegung des Kolbens einer Spritze mit mindestens einer Kanüle, wobei die Spritze auf einem beweglichen Halter angeordnet ist. Für die Erzeugung der Axialschwingungen ist eine Vibrationseinrichtung vorgesehen, die mit dem Halter derart verbunden ist, daß der auf dem Halter angeordneten Injektionsspritze während ihres Vorschubs Axialschwingungen mit einer Amplitude von 0,1 bis 1,5 mm überlagert sind. Der Antrieb und die verstellbar angeordneten Anschläge sind so ausgebildet, daß der Einstich stufig oder mit konstanter Geschwindigkeit einstellbar für eine Tiefe von 3 bis 15 mm in einer Zeit bis zu annähernd 20 s ausführbar ist. Diese Injektionseinrichtung ist für die Selbstinjektion von Pharmaka geeignet und insbesondere auch für die Penis-Stimulation anwendbar.

Diese Injektionseinrichtung weist jedoch einigen Nachteile auf. So ist das Entlüften der Spritze in der Einrichtung nicht möglich, sondern muß vorher von Hand erfolgen, wobei größte Vorsicht beim Einsetzen in die Injektionseinrichtung geboten ist. Der Injektionsvorgang ist nur bezüglich Einstich und Injektion automatisiert; ein selbsttätiges Zurückziehen erfolgt nicht. Die Einstich- und die Kolbenbewegung der Spritze verlaufen ferner, technisch bedingt, gleich schnell. Besser sind jedoch ein schneller Einstich und eine langsame Injektion. Da beide Bewegungen über den Druck auf den Kolben der Injektionsspritze hervorgerufen werden, kann nicht ausgeschlossen werden, daß während des Einstiches bereits eine wenig injiziert wird. Der prinzipielle Aufbau des Vibrations- und seines Übertragungsmechanismus ist ungünstig. Neben den Kanülen werden die Spritzen mit Halterung sowie weitere Massen zum Schwingen erregt, was einen sehr schlechten Wirkungsgrad bedingt und zur Virbation des Gehäuses führt. Außerdem führen die Kanülen aufgrund der genannten Massenbeschleunigungen sowie der Unwucht des Exzenterantriebes radiale Schwingungen aus, die sich als schmerzhaft nachweisen lassen. Da die Spritze schließlich von einer Gewindespindel bewegt wird, mit der sie quasi starr über eine Spindelmutter verbunden ist, wird die Vibration nur aufgrund des Spiels der Spindelmutter übertragen, was zu undefinierbaren Verhältnissen und Einschränkungen bezüglich Amplitude und Frequenz führt.

Der Erfindung, wie sie in den Ansprüchen beschrieben ist, liegt die Aufgabe zugrunde, eine Injektionseinrichtung, insbesondere zur Selbstinjektion von Pharmaka, anzugeben, mit der eine mindestens schmerzarme Injektion und definierten Bedingungen ausführbar ist; vor allem sollen ein rasches Einstechen einer oder mehrerer Kanülen und eine relativ langsame Injektion des Pharmakons gewährleistet sein. Ein besonderer Anwendungsfall für die schmerzarme und definierte Injektion soll dabei die Injektion von Pharmakon in den Penis zum Zwecke seiner Stimulation sein.

Diese Aufgabe wird gemäß der Erfindung durch die Merkmale der Ansprüche 1, 12 und 13 gelöst. Die abhängigen Ansprüche beziehen sich auf zweckmäßige Weiterbildungen des Erfindungskonzepts.

Bei der erfindungsgemäßen Injektionseinrichtung sind den Kanülen nur definierte, axiale Schwingungen, insbesondere hoher Frequenz, überlagert, wobei zugleich ein Mitschwingen der gesamten Injektionseinrichtung bzw. einzelner Baugruppen und eine vorzeitige Injektion während des Einstechens mit Sicherheit ausgeschlossen sind.

Die erfindungsgemäße automatische Injektionseinrichtung hat den Vorteil, daß sie sowohl dem medizinischen Personal als auch dem Patienten bei Selbstinjektion eine wirklich sichere und schmerzarme Anwendung ermöglicht. Durch die Trennung des Antriebsmechanismus für den Einstich der Kanülen und für die Injektion des Pharmakons ist es möglich, einen schnellen, schmerzarmen Einstich vorzunehmen und anschließend eine langsame bzw. in der Geschwindigkeit einstellbare Injektion durchzuführen. Eine vorzeitige Injektion eines geringen Teils des Pharmakons bereits während des Einstiches ist damit ausgeschlossen. Ebenso kann beim Zurückziehen der Kanülen kein Pharmakon wieder eingezogen werden. Die Vibrationseinrichtung, die auf einem massear men Schwingankersystem basiert, wirkt nur auf die Kanülenaufnahme mit den Kanülen. Das elastische Glied zwischen Kanülenaufnahme und Spritzenbehälter sorgt für eine sichere Schwingungstrennung, so daß nur die unbedingt notwendigen Teile, die nur einen äußerst geringen Anteil der Gesamtmasse darstellen, in Schwingung versetzt werden. Dadurch führen nur die Kanülen definierte, axiale Schwingungen hoher Frequenz aus, wobei die Injektionseinrichtung selbst unbeeinflußt bleibt. Schmerzhafte Radialschwingungen treten nicht mehr auf. Der Wirkungsgrad der Vibrationseinrichtung ist hoch.

Die Entlüftung der Spritze erfolgt nach dem Einsetzen in die Injektionseinrichtung. Bei Betätigung des Hebels an der Spritzenaufnahme wird die Entlüftung automatisch durchgeführt. Damit ist garantiert, daß die Spritze vor der Injektion sicher entlüftet ist.

Zur Verwendung zur Penis-Stimulation durch Injektion bekannter Pharmaka bzw. Wirkstoffe in die Schwellkörper des Penis wird vorzugsweise eine Injektionseinrichtung mit zwei Kanülen verwendet. Der Abstand der Kanülen sowie die einstellbare Einstichtiefe werden entsprechend den anatomischen Verhältnissen des Benutzers bestimmt. Für die erfindungsgemäße Injektionseinrichtung sind daher für diesen und ähnliche Einsatzfälle zweckmäßigerweise Kanülenaufnahmen mit verschiedenen Abständen der Kanülen vorgesehen.

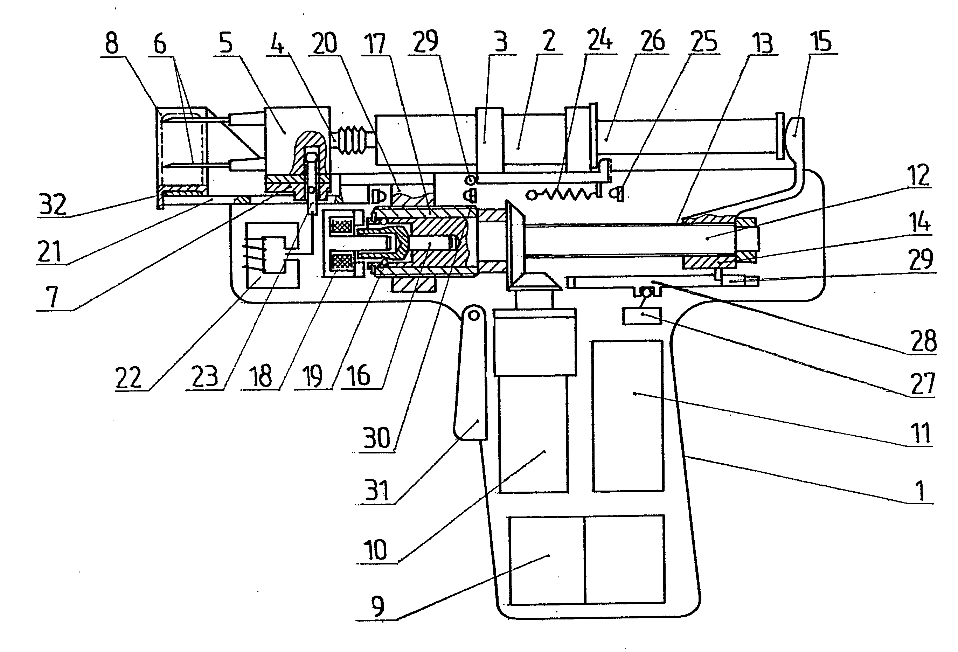

Die Erfindung wird nachstehend anhand eines Ausführungsbeispiels näher erläutert. In der Zeichnung ist der Gesamtaufbau einer erfindungsgemäßen Injectionseinrichtung schematisch dargestellt, die in einem pistolenformigen Gehäuse 1 untergebracht ist. In dem Gehäuse 1 ist im oberen Teil eine Injektionsspritze 2 in einer Spritzenaufnahme 3 gehaltert. Nach dem Füllen der noch nicht aufgestechten Injektionsspritze 2 wird diese zuerst mit einem elastischen Glied 4 und einer Kanülenaufnahme 5, die mit einer oder mehreren Kanülen 6 bestückt ist, komplettiert. Danach wird das Ganze auf die Spritzenaufnahme 3 und einen Schlitten 7 aufgesteckt und ein U-förmiges Mundstück 8 an die Injektionseinrichtung angerastet. Aus Sterilitätsgründen ist des zweckmäßig, außer der Injektionsspritze 2 die Kanülenaufnahme 5, die Kanülen 6, das elastische Glied 4 sowie eventuell das Mündstück 8 als Wegwerfteile vorzusehen, wobei Kanülen 6 und Kanülenaufnahme 5 ein Teil sein kann.

Im Griff des pistolenförmigen Gehäuses 1 befinden sich eine Stromversorgungseinrichtung mit den Batterien 9, ein Gleichstrom-Getriebemotor 10 sowie ein elektronischer Schaltungsteil 11. Der Gleichstrom-Getriebemotor 10 treibt über zwei Kegelräder eine Spindel 12 mit etwa einer Umdrehung pro Sekunde an. Die Spindel 12 ist im hinteren Teil mit einem Gewindestück 13 niedriger Steigung und mit der dazugehörigen Spindelmutter 14, die einen Kolbenschieber 15 bewegt, ausgeführt. Der vordere Spindelteil nimmt eine als Konuskupplung ausgebildete schaltbare Kupplung 16 auf, die im angezogenen Zustand eine Gewindebuchse 17 mit der Spindel 12 koppelt. Der Konus der Kupplung 16 wird zum Einstechen der Kanülen 6 durch Einschalten eines Elektromagneten 18 angezogen, wodurch sich die Kugeln 19 zwischen Spindel 12 und Gewindebuchse 17 verklemmen. Die Gewindebuchse 17 bestizt eine gegenläufige, große Steigung. Die dazugehörige Spindelmutter 20 transportiert einen starr verbundenen Träger 21 einschließlich einer Mundstückaufnahme 32 und aufgestecktem Mundstück 8. Der Einstich der Kanülen 6 in den Körper erfolgt also durch Zurückbewegen des auf der entsprechenden Hautpartie ruhenden Mundstückes 8, das die im Ausgangszustand mechänisch geschützten Kanülen 6 bis zur erforderlichen Einstichtiefe freigibt.

Die Injektionsspritze selbst bzw. der Spritzenbehälter 2 ist in der Spritzenaufnahme 3, außer beim Entlüften, als im Gehäuse 1 ruhend zu betrachten.

Zum schmerzarmen Einstechen der Kanülen 6 ist diesen eine Axialschwingung überlagert. Die dazu erforderliche Vibrationseinrichtung 22 basiert auf einem massearmen Schwingankersystem, das über einen Hebel 23 den gut gleitenden, ausschließlich in Kanülenlängsrichtung beweglichen Schlitten 7 mit aufgesteckter Kanülenaufnahme 5 in Schwingungen mit einer maximalen Amplitude von 0,15 mm versetzt. Das Schwingankersystem wird von dem elektronischen Schaltungsteil 11 angesteuert, der eine variable Frequenz zwischen 0,1 und 1 kHz mit veränderbarer Amplitude erzeugt. Das zwischen dem Spritzenbehälter 2 der Injektionsspritze und der Kanülenaufnahme 5 angeordnete elastische Glied 4 verhindert ein Mitschwingen der Injektionsspritz sowie weiterer Elemente einschließlich des Gehäuses 1. Damit ist auch ein guter Wirkungsgrad der Vibrationseinrichtung 22 garantiert. Schmerzhafte radiale Schwingungen der Kanülen 6 treten nicht auf.

Das Entlüften der aufgesteckten Injektionsspritze erfolgt bei senkrecht nach oben gehaltener Injektionseinrichtung durch geringe Zurückziehen der Spritzenaufnahme 3 mittels eines Hebels gegen die Federkraft einer Zugfeder 24. Dabei startet ein Schaltkontakt 25 den Getriebemotor 10, wodurch der Kolbenschieber 15 bewegt und die Injektionsspritze so lange entlüftet wird, bis der Hebel losgelassen wird. Die axial bewegliche Spritzenaufnahme 3 gleitet nun durch die Zugfeder 24 von selbst wieder in die Ausgangslage zurück.

Dabei entsteht zwischen dem Kolben 26 und dem Kolbenschieber 15 ein funktionell notwendiger Spalt. Dieser Spalt ist erforderlich, da während des Einstiches der Kanülen 6, d.h. also während des durch die Spindeldrehung bewirkten Zurückziehens des Mundstücks 8, der Kolbenschieber 15 zwangsläufig langsam mitgeführt wird, eine vorzeitige Injektion jedoch nicht erfolgen darf. Die Breite des Spaltes bzw. der Abstand zwischen dem Schaltkontakt 25 und dem betätigenden Anschlag entspricht der Zeit, in der das Einstechen der Kanülen 6 erfolgt sein muß.

Für den Getriebemotor 10 ist ein Polwendeschalter 27 vorgesehen, der durch den von der Spindelmutter 14 zwangsgeführten Steuerschieber 28 betätigt wird. Der Steuerschieber 28 ist mit einem verstellbaren Anschlag 29 versehen, der nach der Umschaltung des Getriebemotors 10 den Polwendeschalter 27 wieder in die Ausgangslage kippt. Des weiteren sind zwei Endschalter 30 vorgesehen, die von der Spindelmutter 20 betätigt werden und die Kupplung 16 ausschalten. Am Griff des pistolenförmigen Gehäuses 1 ist ein Schalter 31 angebracht, der den Getriebemotor 10, die Vibrationseinrichtung 22 und den Elektromagneten 18 der Kupplung 16 einschaltet.

Zur Durchführung einer Injektion wird die Injektionsspritze gefüllt und mit dem elastischen Glied 4 und der Kanülenaufnahme 5 mit Kanülen 6 versehen, worauf des Ganze auf die Spritzenaufnahme 3 und den Schlitten 7 aufgesteckt wird. Das Mundstück 8 wird an die Mundstückaufnahme 32 angerastet. Nun wird die Entlüftung der Injektionsspritze vorgenommen, indem der Hebel an der Spritzenaufnahme 3 zurückgezogen wird, wobei der Schaltkontakt 25 den Getriebemotor 10 anschaltet. Im Ausgangszustand hat der Polwendeschalter 27 durch den von der Spindelmutter 14 zwangsgeführten Steuerschieber 28 den Getriebemotor 10 für den Vorwärtslauf vorbereitet. Da der Elektromagnet 18 der Kupplung 16 stromlos bleibt, wird nur der Kolbenschieber 15 bewegt, bis die Injektionsspritze entlüftet ist und der Hebel der Spritzenaufnahme 3 losgelassen wird. Die Spritzenaufnahme 3 wird dann durch die Zugfeder 24 wieder in die Ausgangsstellung bewegt, wobei der funktionell notwendige Spalt zwischen Kolbenschieber 15 und Kolber 26 entsteht. Die Einrichtung ist jetzt für die Injektion vorbereitet, und das Mundstück 8 wird an der entsprechenden Stelle des menschlichen oder tierischen Körpers aufgesetzt. Mit der Betätigung des Griffschalters 31 läuft der Getriebemotor 10 wieder an, und gleichzeitig werden die als Schwingankersystem ausgebildete Vibrationseinrichtung 22 und der Elektromagnet 18 der Kupplung 16 angeschaltet. Die Vibrationseinrichtung 22 versetzt über den Hebel 23 die Kanülenaufnahme 5 mit den Kanülen 6 in Axialschwingungen, wodurch ein schmerzfreier Einstich möglich ist. Durch die angezogene Kupplung 16 wird die Gewindebuchse 17 von der Spindel 12 mitbewegt und das Mundstück 8 von der Spindelmutter 20 über den Träger 21 zurückgezogen. Das Zurückziehen und somit der Einstick der Kanülen 6 erfolgen aufgrund der großen Gewindesteigung rasch. Mit der Drehung der Spindel 12 wird gleichzeitig der Kolbenschieber 15 mitbewegt, jedoch wegen der geringen Steigung wesentlich langsamer. Da ein ausreichend bemessener Spalt zwischen Kolben 26 und Kolbenschieber 15 vorhanden ist, drückt dieser während des Einstiches noch nicht auf den Kolben 26, so daß keine vorzeitige Injektion erfolgen kann. In der Zeit, in der der dem Spalt entsprechende Weg vom Kolbenschieber 15 zurückgelegt wird, muß also der Einstich erfolgt sein. Erreicht die Spindelmutter 20 den hinteren Endschalter 30, werden die Vibrationseinrichtung 22 und der Elektromagnet 18 der Kupplung 16 abgeschaltet. Nun wird der Getriebemotor 10 bei weiterer Betätigung des Griffschalters 31 von einer elektronischen Intervallschaltung des Schaltungsteils 11 gespeist, die durch Veränderung des Tastverhältnisses eine variierbare mittlere Injektionsgeschwindigkeit realisiert. Das Ende der Injektion wird durch den Steuerschieber 28 ausgelöst, der den Polwendeschalter 27 umkippt, worauf der Getriebemotor 10 rückwärts läuft und die Kupplung 16 durch den Elektromagneten 18 eingeschaltet wird. Dadurch wird das Mündstück 8 nach vorn geschoben, und die Kanülen 6 werden aus dem Körper gezogen. Erreicht die Spindelmutter 20 den vorderen Endschalter 30, wird die Kupplung 16 wieder abgeschaltet.

Die automatisch ablaufende Rückzugsbewegung wird durch den Steuerschieber 28 unterbrochen, wenn der Anschlag 29 erreicht ist, der den Polwendeschalter 27 wieder in die Ausgangslage kippt.

Eine Verstellbarkeit und Skalierung des Anschlags 29 verbessert die Dosierung kleinerer Injektionsmengen durch kleine Kolbenhübe der Injektionsspritze. Zur individuellen Anpassung der Injektionsvorrichtung an den Patienten bezüglich der Einstichtiefe und bei Mehrfachspritzen des Einstichabstandes können die Kanülenaufnahmen 5 mit unterschiedlicher Länge und Kanülenabstand sowie die Mundstücke mit unterschiedlicher Länge ausgeführt sein.

Die im Ausführungsbeispiel detailliert dargestellte Doppelspritze mit ihren Einstellmöglichkeiten für Kanülenabstand und Einstichtiefe ist für den besonderen Fall der Injektion von Pharmakon in den Penis gestaltet. In diesem Anwendungsfall kommen als Pharmaka bevorzugt solche Präparate zum Einsatz, die zur Gruppe der arterienerweiternden und venenverengenden Medikamte gehören, wie z.B. Papaverin, Regitin und NaCl-Lösung in bestimmtem Mitschungsverhältnis. Abgesehen von diesem speziellen Anwendungsfall ist die erfindungsgemäße Injektionseinrichtung vor allem auch für die Insulin-Injektion verwendbar, wobei dann nur eine Kanüle auf die Injektionsspritze aufgesetzt ist.

## Ansprüche

1. Injektionseinrichtung mit

a) einer auf einer Spritzenaufnahme (3) angeordneten Injektionsspritze, die aus Spritzenbehälter (2), Kolben (26) und Kanülenaufnahme (5) mit mindestens einer Kanüle (6) besteht,

b) einem Antrieb (10, 12) für die Bewegung des Kolbens (26) sowie für das Einstechen und Zurückziehen der Kanülen (6), der
- einerseits mit einem nur für die Injektion vorgesehenen Kolbenschieber (15) und
- andererseits über eine schaltbare Kupplung (16) mit einem axial beweglichen, die Kanülen (6) freigebenden Mundstück (8) in Verbindung steht,

c) einer Vibrationseinrichtung (22), die mit der Kanülenaufnahme (5) verbunden ist und zumindest während des Einstiches den Kanülen (6) Axialschwingungen überlagert,
und

d) einem elastischen, die Vibration nicht übertragenden Glied (4) zwischen Spritzenbehälter (2) und Kanülenaufnahme (5).

2. Injektionseinrichtung nach Anspruch 1, dadurch gekennzeichnet, daß die axial bewegliche Spritzenaufnahme (3) zur Entlüftung zurückziehbar ist, in dieser Stellung der Antrieb (10, 12) anläuft und die Spritzenaufnahme (3) unter Bildung eines Spaltes zwischen Kolben (26) und Kolbenschieber (15) wieder in die Ausgangsstellung zurückgleitet.

3. Injektionseinrichtung nach Anspruch 1 oder 2, dadurch gekennzeichnet, daß der Antrieb von einem Getriebemotor (10) gebildet ist, der mit einer Spindel (12) in Verbindung steht, die mit einem Gewindestück (13) niedriger Steigung für die Bewegung des Kolbenschiebers (15) versehen und über die Kupplung (16) mit einer Gewindebuchse (17) großer Steigung für die Bewegung des Mundstückes (8) verbindbar ist.

4. Injektionseinrichtung nach einem der Ansprüche 1 bis 3, dadurch gekennzeichnet, daß die Kupplung (16) eine Konuskupplung ist, die von einem Elektromagneten (18) betätigbar ist.

5. Injektionseinrichtung nach einem der Ansprüche 1 bis 4, dadurch gekennzeichnet, daß die Vibrationseinrichtung (22) als massearmes Schwingankersystem ausgeführt ist, das von einem elektronischen Schaltungsteil (11) bis zu Amplituden von 0,15 mm und in der Frequenz im Bereich von 0,1 bis 1 kHz veränderlich ansteuerbar ist.

6. Injektionseinrichtung nach einem der Ansprüche 1 bis 5, gekennzeichnet durch zwei Endschalter (30), die mit der Bewegung des Mundstücks (8) betätigbar sind, und einen Polwendeschalter (27) für den Getriebemotor (10), der mit der Bewegung des Kolbenschiebers (15) über einen zwangsgeführten Steuerschieber (28) betätigbar ist.

7. Injektionseinrichtung nach einem der Ansprüche 1 bis 6, dadurch gekennzeichnet, daß zur Dosierung der Injektionsmengen am Steuerschieber (28) ein verstellbarer Anschlag (29) mit Skalierung angebracht ist.

8. Injektionseinrichtung nach einem der Ansprüche 3 bis 7, dadurch gekennzeichnet, daß der Getriebemotor (10) von einer elektronischen Intervallschaltung des elektronischen Schaltungsteils (11) mit veränderlichem Tastverhältnis zur Realisierung verschiedener, extrem kleiner Injektionsgeschwindigkeiten angesteuert ist.

9. Injektionseinrichtung nach einem der Ansprüche 1 bis 8, dadurch gekennzeichnet, daß die Einstichtiefe der Kanülen (6) durch unterschiedliche Längen der Mundstücke (8) bzw. der Kanülenaufnahme (5) variierbar ist.

10. Injektionseinrichtung nach einem der Ansprüche 1 bis 9, dadurch gekennzeichnet, daß die Injektionsspritze (2), das elastische Glied (4), die Kanülenaufnahme (5) mit Kanülen (6) und/oder das Mündstück (8) als Wegwerfteile ausgeführt sind.

11. Injektionseinrichtung nach einem der Ansprüche 1 bis 10, dadurch gekennzeichnet, daß Kanülenaufnahmen (5) mit zwei Kanülen (6) mit verschiedenem, benutzerspezifischem Abstand verwendbar sind.

12. Verwendung der Injektionseinrichtung nach einem der Ansprüche 1 bis 10 mit einer einzigen Kanüle (6) zur Injektion von Insulin.

13. Verwendung der Injektionseinrichtung nach einem der Ansprüche 1 bis 11 mit zwei Kanülen (6) zur Penis-Stimulation.

EP 0 302 278 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,X | EP-A-0 239 721 (VEB TRANSFORMATOREN- UND RÖNTGENWERK "H. MATERN") * Beschreibung; Figuren * --- | 1-13 | A 61 M 5/20 |
| D,A | DD-A- 132 169 (PISTOR) ----- | | |

|  | RECHERCHIERTE SACHGEBIETE (Int. Cl.4) |
|---|---|
|  | A 61 M |

Der vorliegende Recherchenbericht wurde für alle Patentansprüche erstellt

| Recherchenort | Abschlußdatum der Recherche | Prüfer |
|---|---|---|
| DEN HAAG | 10-11-1988 | VANRUNXT J.M.A. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

...............................................................................................

& : Mitglied der gleichen Patentfamilie, übereinstimmendes Dokument